# FASCICULE DE BREVET EUROPEEN

(11) **EP 2 460 594 B1**
(45) Date de publication et mention de la délivrance du brevet: **28.02.2018**
(21) Numéro de dépôt: 11306579.1
(22) Date de dépôt: 29.11.2011
(51) Int. Cl.: B09B 3/00, B03B 9/06, C05F 17/00

(54) **Procédé et dispositif de tri et de traitement de déchets ménagers bruts**
Verfahren und Vorrichtung zur Abfallsortierung und -aufbereitung von unsortiertem Haushaltsmüll
Method and device for sorting and treating raw household waste

(30) Priorité: 01.12.2010 FR 1059980
(43) Date de publication de la demande: 06.06.2012
(73) Titulaire: Pena Environnement, 33127 Saint Jean D'Illac (FR)
(72) Inventeur: Pena, Marc, 33610 CANEJAN (FR)
(74) Mandataire: Aquinov

(56) Documents cités:
- EP-A2- 1 310 307
- WO-A1-03/078084
- US-A- 4 874 134

## Description

La présente invention concerne un procédé et un dispositif pour le tri et pour le traitement de déchets ménagers bruts.

On sait traiter de façon séparée les différentes composantes des déchets ménagers bruts.

Le problème reste de pouvoir assurer un traitement des déchets bruts, directement collectés, sans effectuer un tri préalable.

En effet, il faut tenir compte des coûts et on imagine que, s'il faut préalablement assurer un tri, le coût du traitement est augmenté d'autant.

Afin de conduire à un traitement de l'ensemble des déchets et du recyclage, il faut en effet que le coût du retraitement soit incitatif et non dissuasif.

Il existe déjà des systèmes permettant de traiter des déchets ménagers bruts. On connaît ainsi le cylindre DANO® qui est un cylindre tournant, de grandes dimensions, susceptible de recevoir les ordures, en brut.

Ce cylindre peut atteindre plusieurs dizaines de mètres de long, entre 25 et 40m de long et 2 à 4m de diamètre, pour certains d'entre eux.

Un tel cylindre peut traiter des quantités de 100 tonnes/jour de déchets ménagers suivant les types de déchets.

Le cylindre est incliné avec une très faible pente et rotatif et se trouve empli entre 50 et 80%. La vitesse de rotation est très faible, 0,1 à 2 tours par min. Les déchets se déplacent de manière hélicoïdale vers la sortie simultanément avec la rotation du réacteur et l'inclinaison, la durée de séjour variant de 1 à 4 jours. Durant ce parcours, les déchets sont homogénéisés et subissent, par abrasion, une réduction de la granulométrie.

Les résidus ainsi obtenus sont criblés.

Par contre, si ce procédé est simple, il génère des odeurs.

On note aussi une quantité d'énergie importante nécessaire pour faire tourner ce tambour ainsi qu'un investissement financier initial important.

Surtout, le traitement des déchets est très limité et peu efficace.

En effet, la matière organique conduit à la formation de boulettes de type pâte à papier et comme le verre est broyé, que le sable est conservé et que les salissures ne sont pas éliminées, elles se retrouvent dans lesdites boulettes, limitant leur emploi en compostage.

La bactériologie susceptible de dégrader la matière organique se développe difficilement et surtout elle est différente de ce qu'elle devrait être du fait de la présence de matières autres qu'organiques.

Le produit résultant est difficilement conforme aux normes en vigueur.

Il est à noter que les métaux des piles sont aussi mélangés.

Quant aux contenants en métal ils sont souvent remplis de cette matière pâteuse, interdisant le recyclage de ces contenants et conduisant souvent à leur enfouissage.

Ce système ne donne donc pas satisfaction.

Le document US 4 874 134 décrit un dispositif qui prévoit un tri de déchets en début de procédé et un broyage ensuite. Un cyclonage en enceinte fermée permet de séparer le papiers et les particules organiques, les plastiques ayant été retirés en tête de procédé, manuellement. Les particules organiques sont collectées.

Le procédé et le dispositif selon la présente invention propose le tri des matières organiques brutes, directement en sortie des véhicules collecteurs.

Le procédé est d'un coût permettant de conduire à une rentabilité et non une dépense pour l'élimination, grâce à la valorisation du compost obtenu.

Le procédé selon la présente invention est maintenant décrit en détail suivant un mode de réalisation particulier, non limitatif.

Sur les dessins annexés, les figures représentent :
- Figure 1 : une vue du synoptique du procédé, et
- Figure 2 : une vue du dispositif associé.

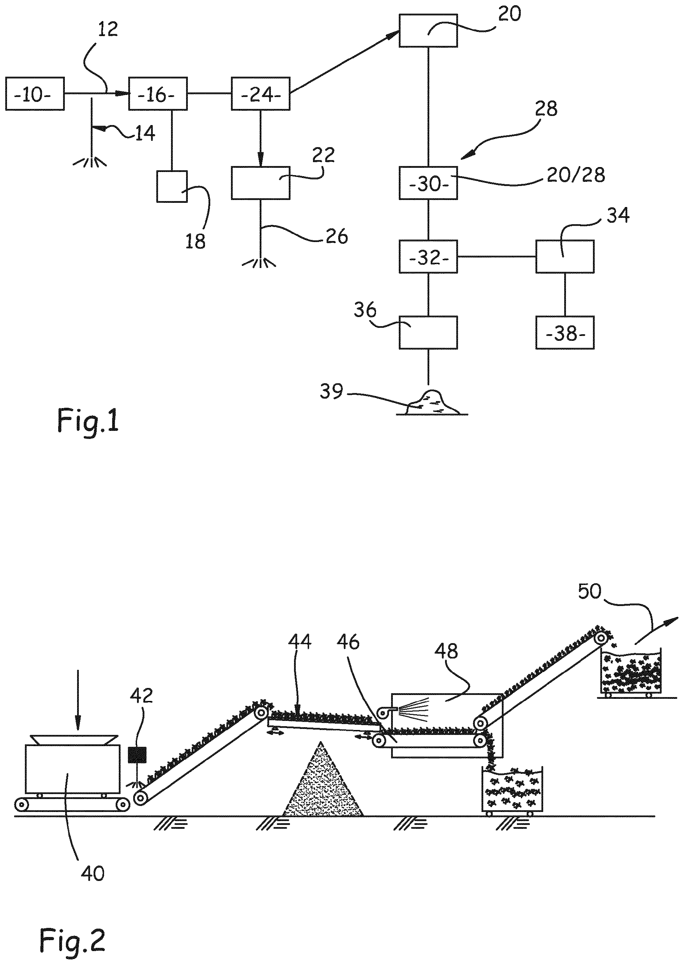

Le procédé selon la présente invention consiste à recevoir les déchets bruts dans une trémie, comme l'indique le synoptique de la figure 1.

Ces déchets sont constitués de :
- Fines : poussières, sables, petite particules
- Lourds : ferrailles, verre, contenants métalliques, stériles (cailloux, verre, porcelaine), toxiques (piles)
- Matière organique : déchets alimentaires
- Matières plastiques.

La première étape 10 du procédé est un broyage grossier en morceaux de l'ordre de 10 cm pour donner un ordre de grandeur pour donner une matière 12 grossièrement broyée.

En sortie de l'étape de broyage, les éléments métalliques magnétiques sont retirés de la matière 12 grossièrement broyée, au cours d'une étape 14.

L'étape suivante 16 consiste à assurer un tamisage des matières grossièrement broyées desquelles les éléments métalliques ont été retirés.

Le tamisage est à une maille d'environ 5 à 20 mm laissant ainsi passer les fines 18 de dimensions inférieures.

Les fines 18 ne sont pas recyclables et incluent les graviers, les sables et les fragments de verre, de porcelaine, les coquillages, les fragments de polystyrène. Ces fragments de polystyrène sont résistants, solides et de petites dimensions au point de ne pas se lier aux autres composants et de se comporter comme des particules isolées, libres.

Ces fines issues de ce tamisage sont mises en décharge. Elles représentent 10 à 15% en poids des déchets bruts.

Le refus doit être traité pour distinguer la fraction 20 compostable, composée des matières plastiques, papiers, cartons et matières purement organiques de la fraction 22 des déchets lourds.

Cette étape 24 est dite de séparation aéraulique.

Elle consiste à souffler dans un tunnel de l'air avec une vitesse importante du flux d'air.

Les matières légères constituant la fraction légère sont emportées par le flux d'air et les matières lourdes constituent le refus.

La matière légère est la fraction 20 compostable et représente environ 80% des déchets bruts.

Le refus lourd constitue la fraction 22 des déchets lourds, représentant environ 7%.

Cette fraction de déchets lourds est évacuée et les éléments ferromagnétiques qui peuvent subsister sont retirés par passage sur une bande dite Overband avec des têtes magnétiques qui retiennent les pièces ferromagnétiques telles que boîtes de conserve, et couvercles ainsi que les emballages liés aux métaux ferreux, étape 26.

On obtient ainsi une sous séparation de la fraction lourde des déchets avec :
- environ 5% en flacons de parfum ou de médicaments, fruits et légumes entiers, bouteilles en verre, cailloux, emballages lourds, chaussures, vaisselle cassée pour indiquer une liste non exhaustive.
- Environ 2% de contenants ferromagnétiques, boîtes de conserve, couvercles. Ces sous-produits sont recyclables et proviennent essentiellement de la première étape de collecte en sortie du broyeur.

Le procédé selon l'invention propose un traitement 30 de la fraction 20 compostable qui comprend une fermentation en enceinte close.

Ce compostage consiste à adjoindre à la fraction 20 légère des déchets verts et/ou des boues de station d'épuration ou autres déchets organiques qui agissent comme des structurants 28.

Le traitement consiste en un stockage statique de cette fraction légère structurée 20/28 compostable, en enceinte close avec apport d'air et donc d'oxygène pour le développement de microorganismes aptes à assurer la transformation des matières organiques.

La température de fermentation monte à 70°C de sorte à permettre un développement de la faune bactérienne souhaitée, sans émanation d'odeurs, cette température permettant une hygiénisation du produit.

La durée est d'environ une quinzaine de jours pour donner un ordre de grandeur. Durant cette phase de fermentation, il est prévu des retournements du volume ainsi stocké de façon à assurer une homogénéisation du volume traité.

Le résultat est un mélange de particules de matière plastique mélangées à du compost.

Un tamisage lors d'une dernière étape 32 permet de séparer les particules de compost 34 qui passe par gravité à travers un tamis de 5 à 20 mm tandis que les particules 36 de matière plastique sont retenues.

Le compost 34 est alors maturé, étape 38, pour le rendre directement utilisable par l'agriculture.

Les particules 36 de matière plastique sont granulées, étape 39, pour servir de combustible.

Ces particules 36 de matière plastique sont utilisées pour l'alimentation de fours ou de chaudières industrielles, présentant un très fort pouvoir calorifique du fait de l'absence d'autres déchets.

Ainsi, à partir de déchets ménagers bruts, on obtient :*
- Des fines à mettre en décharge 10 à 15%
- Des déchets ferromagnétiques 2%
- Des déchets lourds à éliminer 5%
- De la matière combustible 35 à 50%
- Du compost 15 à 20% en adjoignant 0,5 à 1,5 tonnes de déchets verts/boues par tonne de déchets, en brut.

Le dispositif comprend un broyeur 40 de pré broyage grossier en éléments de taille d'une dizaine de centimètres notamment par passage à travers un pré broyeur commercialisé sous la marque Terminator.

Les moyens 42 de tri des déchets ferromagnétiques en sortie de pré broyeur comprennent un système dit Overband et un tambour magnétique.

Le tamis 44 des fines est du type crible horizontal à paliers.

Un convoyeur 46 incliné permet de transférer la matière brute broyée grossièrement jusqu'à un séparateur 48 aéraulique.

Quant aux moyens 50 pour le traitement du compost, on peut se reporter à la demande EP 1486478 A1, au nom du même demandeur, qui décrit dans le détail un mode de mise en oeuvre des étapes de fabrication d'un compost uniquement à base de déchets verts et boues de station d'épuration.

## Revendications

1. Procédé de traitement de déchets ménagers bruts, qui comprend les étapes suivantes réalisées dans l'ordre suivant :
- Broyage (10) grossier desdits déchets,
- Retrait des matières ferromagnétiques (14) des déchets broyés,
- Tamisage (16) des fines à partir des déchets broyés desquels les éléments ferromagnétiques ont été retirés,
- Séparation (24) aéraulique du refus en une fraction lourde (22) et une fraction légère,
- Traitement de la fraction (22) lourde, et
- Traitement de la fraction (20) légère par compostage en enceinte close séparation (32) des particules de matière plastique et des particules de compost.

2. Procédé de traitement de déchets ménagers bruts selon la revendication 1, **caractérisé en ce que** l'étape de compostage en enceinte close consiste en la succession des étapes suivantes :
- Adjonction à la fraction légère (20) de déchets verts et/ou de boues de station d'épuration ou autres déchets organiques,
- Fermentation en enceinte close de l'ensemble fraction légère/déchets verts et/ou de boues de station d'épuration, à une température de 70°C
- Séparation des particules (36) de matière plastique et des particules (34) de compost.

3. Procédé de traitement de déchets ménagers bruts selon la revendication 2, **caractérisé en ce que** la séparation des particules de matière plastique et des particules de compost est réalisée par tamisage.

4. Procédé de traitement de déchets ménagers bruts selon la revendication 2 ou 3, **caractérisé en ce que** les particules (36) de matière plastique sont granulées.

5. Procédé de traitement de déchets ménagers bruts selon la revendication 2, 3 ou 4, **caractérisé en ce que** les particules de compost (34) subissent une phase de maturation.

6. Procédé de traitement de déchets ménagers bruts selon l'une quelconque des revendications précédentes, **caractérisé en ce que** l'on retire les éléments ferromagnétiques qui peuvent subsister dans la fraction (22) lourde.

7. Dispositif pour la mise en oeuvre du procédé selon l'une quelconque des revendications précédentes, qui comprend au moins dans l'ordre suivant :
- Un broyeur (40) grossier de la matière brute
- Des moyens (42) de tri des déchets ferromagnétiques en sortie de broyeur
- Un tamis (44) de séparation des fines,
- Un convoyeur (46) incliné,
- Un séparateur (48) aéraulique de la fraction lourde et de la fraction légère,
- Des moyens de traitement de la fraction lourde (22),
- Des moyens de traitement de la fraction légère (20) par compostage,
- des moyens de séparation (32) des particules de matière plastique et des particules de compost.

## Patentansprüche

1. Verfahren zur Verarbeitung von unsortiertem Haushaltsmüll, das die folgenden, in der folgenden Reihenfolge ausgeführten Verfahrensschritte aufweist:
- Grobzerkleinerung (10) der Abfälle,
- Entfernen von ferromagnetischen Materialien (14) aus den zerkleinerten Abfällen,
- Absieben (16) von Feinteilen ausgehend vom zerkleinerten Abfall, aus dem die ferromagnetischen Elemente entfernt worden sind,
- Trennung (24) mittels Luftströmung des Rückstands in einen schweren Anteil (22) und einen leichten Anteil,
- Verarbeitung des schweren Anteils (22), und
- Verarbeitung des leichten Anteils (20) durch Kompostierung in einem abgeschlossenen Bereich
- Trennung (32) der Teilchen aus Plastikmaterial und der Kompostteilchen.

2. Verfahren zur Verarbeitung von unsortiertem Haushaltsmüll nach Anspruch 1, **dadurch gekennzeichnet, dass** das Verfahren der Kompostierung in einem abgeschlossenen Bereich aus der Abfolge der nachfolgenden Verfahrensschritte besteht:
- Hinzufügen von Grünabfällen und/oder Klärschlamm oder anderen organischen Abfällen zum leichten Anteil (20),
- Fermentierung des leichten Anteils/der Grünabfälle und/oder des Klärschlamms in einem abgeschlossenen Bereich bei einer Temperatur von 70°C,
- Trennung der Teilchen (36) aus Plastikmaterial und der Kompostteilchen (34).

3. Verfahren zur Verarbeitung von unsortiertem Haushaltsmüll nach Anspruch 2, **dadurch gekennzeichnet, dass** die Trennung der Teilchen aus Plastikmaterial und der Kompostteilchen durch Sieben bewerkstelligt wird.

4. Verfahren zur Verarbeitung von unsortiertem Haushaltsmüll nach Anspruch 2 oder 3, **dadurch gekennzeichnet, dass** die Teilchen (36) aus Plastikmaterial granuliert werden.

5. Verfahren zur Verarbeitung von unsortiertem Haushaltsmüll nach Anspruch 2, 3 oder 4, **dadurch gekennzeichnet, dass** die Kompostteilchen (34) einer Reifephase unterzogen werden.

6. Verfahren zur Verarbeitung von unsortiertem Haushaltsmüll nach einem der vorhergehenden Ansprüche, **dadurch gekennzeichnet, dass** die ferromagnetischen Elemente entfernt werden, die in dem schweren Anteil (22) fortbestehen.

7. Vorrichtung für die Ausführung des Verfahrens nach einem der vorhergehenden Ansprüche, die in der nachfolgenden Reihenfolge wenigstens aufweist:
- einen Grobzerkleinerer (40) des unsortierten Materials,
- ein Sortiermittel (42) der ferromagnetischen Abfälle am Ausgang des Zerkleinerers,
- ein Sieb (44) zum Abtrennen von Feinteilen,
- eine geneigte Förderanlage (46),
- eine Trennanlage (48) mittels Luftströmung für den schweren Anteil und den leichten Anteil,
- ein Verarbeitungsmittel für den schweren Anteil (22),
- ein Verarbeitungsmittel für den leichten Anteil (20) durch Kompostierung,
- ein Trennmittel (32) für die Teilchen aus Plastikmaterial und die Kompostteilchen.

## Claims

1. A method for treating raw household waste, which comprises the following steps performed in the following order:
- coarse grinding of said waste,
- removal of ferromagnetic materials from the ground waste,
- sieving of the fines from the ground waste from which said ferromagnetic elements have been removed,
- aeraulic separation (24) of the retained material into a heavy fraction (22) and a light fraction,
- treatment of the heavy fraction (22), and
- treatment of the light fraction (20) by composting in a closed chamber,
- separation (32) of the particles of plastics material and the particles of compost.

2. A method for treating raw household waste according to claim 1, **characterised in that** the composting step in a closed chamber comprises the succession of the following steps:
- addition of green waste and/or sewerage-plant sludge or other organic waste to the light fraction (20),
- fermentation in a closed chamber of the amalgamation comprising light fraction/green waste and/or sewerage plant sludge, at a temperature of 70°C,
- separation of the particles (36) of plastics material and the particles (34) of compost.

3. A method for treating raw household waste according to claim 2, **characterised in that** the separation of the particles of plastics material and the particles of compost is carried out by sieving.

4. A method for treating raw household waste according to claim 2 or 3, **characterised in that** the particles (36) of plastics material are granulated.

5. A method for treating raw household waste according to claim 2, 3 or 4, **characterised in that** the particles of compost (34) undergo a maturation phase.

6. A method for treating raw household waste according to any of the preceding claims, **characterised in that** any ferromagnetic elements that may remain in the heavy fraction (22) are removed.

7. A device for implementing the method according to any of the preceding claims, which comprises at least, in the following order:
- a coarse grinder (40) for the raw material,
- means (42) for sorting the ferromagnetic waste at the discharge from the grinder,
- a sieve (44) for separating the fines,
- an inclined conveyor (46),
- an aeraulic separator (48) for separating the heavy fraction and the light fraction,
- means for treating the heavy fraction (22),
- means for treating the light fraction (20) by composting,
- means (32) for separating the particles of plastics material and the particles of compost.
